# EUROPEAN PATENT SPECIFICATION

(11) **EP 1 886 669 B1**
(45) Date of publication and mention of the grant of the patent: **22.01.2014**
(21) Application number: 07113670.9
(22) Date of filing: 02.08.2007
(51) Int. Cl.: A61K 9/00, A61K 9/127, A61K 31/16, A61K 31/198, A61K 31/4415, A61K 31/714, A61K 31/716, A61K 33/18, A61K 45/06

(54) **Phosphoglucan based immunotrophic preparation for the treatment of adenotonsillar hypertrophy**
Phosphoglucanbasierte immunotrophische Herstellung zur Behandlung adenotonsillärer Hypertrophie
Préparation immunotrophique basée sur la phosphoglucane pour le traitement de l'hypertrophie adénotonsillaire

(30) Priority: 03.08.2006 IT RM20060421
(43) Date of publication of application: 13.02.2008
(73) Proprietor: D.M.G. Italia Srl, 00040 Pomezia (RM) (IT)
(72) Inventor: Mercuri, Luigi, I-00040, Pomezia (RM) (IT)
(74) Representative: Sarpi, Maurizio

(56) References cited:
- EP-A- 1 195 178
- WO-A-01/85141
- BABINCOVA M ET AL: "ANTIOXIDANT PROPERTIES OF CARBOXYMETHYL GLUCAN: COMPARATIVE ANALYSIS" JOURNAL OF MEDICINAL FOOD, MARY ANN LIEBERT, LARCHMONT, NY, US, vol. 5, no. 2, 1 June 2002 (2002-06-01), pages 79-83, XP009073120 ISSN: 1096-620X

## Description

The present invention relates to the pharmaceutical dietetic sector, and more in particular to a preparation in the form of syrup with a base of a liposomal complex of CM-glucan (carboxymethylated glucan), arginine, and iodine, with the addition of vitamins of group B and optionally amino acids and/or taurine, which is able to combat adenotonsillar hypertrophy in so far as it improves the normal activity of the immune system and favours trophism of the adenotonsillar lymphoid tissue. The preparation is particularly indicated for small children since the presence of L-arginine improves the condition of their growth, favouring the production of growth hormone, which is usually deficient in subjects who suffer from frequent night wakening.

The presence of the liposomal/phosphoglucan complex, i.e., the liposomal complex of sodium carboxymethylbetaglucan, improves the bioavailability in so far as it enables release of the active principle in a controlled and constant way throughout the day. Furthermore, said complex enables reduction in the disadvantages linked to the absorption, at a gastric level, of the active principle in so far as it acts also as gastro-protector.

The European patent No. 1 195 178, filed in the name of the present applicant, describes a preparation in the form of syrup with a base of beta-glucan, arginine, and iodine, with the addition of vitamins of group B, which can be used for the treatment of adenotonsillar hypertrophy, which improves the normal activity of the immune system and favours trophism of the adenotonsillar lymphoid tissue.

The international patent application No. PCT/IT2006/000543, filed in the name of the present applicant, describes the preparation and use of a liposomal complex of CM-glucan in dermatological, oral and ophthalmic applications. In particular, it envisages englobing CM-glucan in transparent phospholipidic vesicles in such a way as to improve the absorption of CM-glucan at a cutaneous level, increasing its availability at a cellular level.

In the light of what is known in the state of the art, the present invention has set itself the task of potentiating the therapeutic effect of the immunotrophic preparation with a base of beta-glucan for the treatment of adenotonsillar hypertrophy.

In fact, it had been found that beta-glucan in the soluble form presented a reduced absorption, especially at a gastric level, and consequently a low availability, and moreover that it could cause phenomena of hypersensitivity of the gastric mucosa.

The present invention enables solution of the aforesaid technical problems by means of the composition as described in Claim 1.

Consequently, forming a subject of the present invention is a composition that can be used for treatment of adenotonsillar hypertrophy comprising as active principles a liposomal complex of CM-glucan (carboxymethylated glucan), arginine, and iodine with the addition of vitamins of group B, preferably B5, B6, B12, optionally comprising also amino acids chosen in the group consisting of: Ala/A, Arg/R, Asn/N, Asp/D, Cys/C, Glu/E, Gln/Q, Gly/G, Ile/I, Leu/L, Lys/K, Met/M, Phe/F, Pro/P, Ser/S, Thr/T, Trp/W, Tyr, Y, Val/V, preferably Lys/K, and optionally also taurine.

Further characteristics of the invention will emerge from the ensuing detailed description.

According to the invention, a preparation is proposed in the form of syrup, in which, in every 100 ml of product, there are present:

| | |
|---|---|
| Arginine | from 4 to 10 g |
| Vitamin B5 | from 48 to 120 mg |
| Vitamin B6 | from 8 to 20 mg |
| Vitamin B12 | from 8 to 20 mcg |
| Iodine | from 1.600 to 4.000 mg |
| Liposomal/phosphoglucan Complex | from 32 to 80 mg |

Optionally, added to the composition are amino acids chosen in the group consisting of: Ala/A, Arg/R, Asn/N, Asp/D, Cys/C, Glu/E, Gln/Q, Gly/G, Ile/I, Leu/L, Lys/K, Met/M, Phe/F, Pro/P, Ser/S, Thr/T, Trp/W, Tyr, Y, Val/V, in a concentration of 4 to 10 g.

Preferably, the amino acid is Lys/K.

Furthermore, optionally added to the composition is taurine in a concentration of 4 to 10 g.

In a preferred embodiment, the preparation, in the form of syrup comprises, for every 100 ml of product:

| | Per 100 ml |
|---|---|
| Proteins | 5 g |
| Carbohydrates | 40.24 g |
| Fats | 0.9 g |
| Arginine | 5 g |
| Vitamin B5 | 60 mg |
| Vitamin B6 | 10 mg |
| Vitamin B12 | 10 mcg |
| Iodine | 2.000 mg |
| Phosphoglucan | 40 mg |
| Deionized water | As required |

Optionally, added to the composition are amino acids chosen in group consisting of: Ala/A, Arg/R, Asn/N, Asp/D, Cys/C, Glu/E, Gln/Q, Gly/G, Ile/I, Leu/L, Lys/K, Met/M, Phe/F, Pro/P, Ser/S, Thr/T, Trp/W, Tyr, Y, Val/V, in a concentration of 5 g.

Preferably, the amino acid is Lys/K.

Furthermore, optionally added to the composition is taurine in a concentration of 5 g.

In a further embodiment, the composition comprises for every 100 ml of product:

| | Per 100 ml |
|---|---|
| Fructose | 28 g |
| Phosphoglucan | 4.4 g |
| Arginine | 5 g |
| Vitamin B5 | 60 mg |
| Vitamin B6 | 10 mg |
| Vitamin B12 | 0.01 mg |
| Potassium iodide | 26 mg |
| Citric acid | 1.8 g |
| Xanthan gum | 70 mg |
| Sodium benzoate | 0.1 g |
| Sodium methyl para-oxybenzoate | 0.1 g |
| Vanilla aroma | 0.35 g |
| Caramel colorant | 0.01 g |
| Deionized water | As required |

Optionally, the composition comprises Lys/K in a concentration of 5 g.

Optionally, the composition further comprises taurine in a concentration of 5 g.

In general, the composition of the present invention can be administered in the form of a single daily dose from 5 to 10 ml.

## Claims

1. A preparation comprising arginine, iodine, vitamins of group B and a liposomal complex of CM-glucan (carboxymethylated glucan).

2. Use of a composition comprising arginine, iodine, a liposomal complex of CM-glucan (carboxymethylated glucan) and vitamins of group B, for the preparation of a medicament for the therapeutic treatment of adenotonsillar hypertrophy.

3. Use of the composition according to Claim 2, **characterized in that** the vitamins of group B are chosen in the group consisting of B5, B6, B12.

4. Use of the composition according to Claim 2, further comprising amino acids chosen in the group consisting of: Ala/A, Arg/R, Asn/N, Asp/D, Cys/C, Glu/E, Gln/Q, Gly/G, Ile/I, Leu/L, Lys/K, Met/M, Phe/F, Pro/P, Ser/S, Thr/T, Trp/W, Tyr, Y, AND Val/V.

5. Use of the composition according to Claim 4, **characterized in that** the amino acid is Lys/K.

6. Use of the composition according to any one of Claims 2 to 5, further comprising taurine.

7. Use of the composition according to Claim 2, **characterized in that** arginine is in a concentration of 4 to 10 g per 100 ml of product.

8. Use of the composition according to Claim 2, **characterized in that** the liposomal complex of CM-glucan (carboxymethylated glucan) is in a concentration of 32 to 80 mg per 100 ml of product.

9. Use of the composition according to Claim 2, **characterized in that** iodine is in a concentration of 1.600 to 4.000 mg per 100 ml of product.

10. Use of the composition according to Claim 3, **characterized in that** vitamin B5 is in a concentration of 48 to 120 mg per 100 ml of product.

11. Use of the composition according to Claim 3, **characterized in that** vitamin B6 is in a concentration of 8 to 20 mg per 100 ml of product.

12. Use of the composition according to Claim 3, **characterized in that** vitamin B12 is in a concentration of 8 to 20 mcg per 100 ml of product.

13. Use of the composition according to Claim 4, **characterized in that** the amino acids chosen in the group consisting of Ala/A, Arg/R, Asn/N, Asp/D, Cys/C, Glu/E, Gln/Q, Gly/G, Ile/I, Leu/L, Lys/K, Met/M, Phe/F, Pro/P, Ser/S, Thr/T, Trp/W, Tyr, Y, Val/V are in a concentration of 4 to 10 g per 100 ml of product.

14. Use of the composition according to Claim 6, **characterized in that** taurine is in a concentration of 4 to 10 g per 100 ml of product.

15. Use of the composition according to Claim 2, having the following composition for every 100 ml of product:
| | |
|---|---|
| Proteins | 5 g |
| Carbohydrates | 40.24 g |
| Fats | 0.9 g |
| Arginine | 5 g |
| Vitamin B5 | 60 mg |
| Vitamin B6 | 10 mg |
| Vitamin B12 | 10 mcg |
| Iodine | 2.000 mg |
| Phosphoglucan | 40 mg |
| Deionized wateras required | |

16. A syrup for the treatment of adenotonsillar hypertrophy having the following composition for every 100 ml of product:
| | |
|---|---|
| Fructose | 28 g |
| Phosphoglucan | 4.4 g |
| Arginine | 5 g |
| Vitamin B5 | 60 mg |
| Vitamin B6 | 10 mg |
| Vitamin B12 | 0.01 mg |
| Potassium iodide | 26 mg |
| Citric acid | 1.8 g |
| Xanthan gum | 70 mg |
| Sodium benzoate | 0.1 g |
| Sodium methyl para-oxybenzoate | 0.1 g |
| Vanilla aroma | 0.35 g |
| Caramel colorant | 0.01 g |
| Deionized water | as required |

## Patentansprüche

1. Zubereitung, enthaltend Arginin, Jod, Vitamine der Gruppe B und einen liposomalen Komplex aus CM-Glucan (carboxymethyliertes Glucan).

2. Verwendung einer Zusammensetzung enthaltend Arginin, Jod, einen liposomalen Komplex aus CM-Glucan (carboxymethyliertes Glucan) und Vitamine der Gruppe B für die Zubereitung eines Arzneimittels zur therapeutischen Behandlung von adenotonsillarer Hypertrophie.

3. Verwendung der Zusammensetzung nach Anspruch 2, **dadurch gekennzeichnet, dass** die Vitamine der Gruppe B ausgewählt sind aus der Gruppe bestehend aus B5, B6, B12.

4. Verwendung der Zusammensetzung nach Anspruch 2, ferner enthaltend Aminosäuren, ausgewählt aus der Gruppe bestehend aus: Ala/A, Arg/R, Asn/N, Asp/D, Cys/C, Glu/E, Gln/Q, Gly/G, Ile/I, Leu/L, Lys/K, Met/M, Phe/F, Pro/P, Ser/S, Thr/T, Trp/W, Tyr, Y, UND Val/V.

5. Verwendung der Zusammensetzung nach Anspruch 4, **dadurch gekennzeichnet, dass** die Aminosäure Lys/K ist.

6. Verwendung der Zusammensetzung nach einem der Ansprüche 2 bis 5, ferner enthaltend Taurin.

7. Verwendung der Zusammensetzung nach Anspruch 2, **dadurch gekennzeichnet, dass** Arginin in einer Konzentration von 4 bis 10 g pro 100 ml Erzeugnis vorliegt.

8. Verwendung der Zusammensetzung nach Anspruch 2, **dadurch gekennzeichnet, dass** der liposomale Komplex aus CM-Glucan (carboxymethyliertes Glucan) in einer Konzentration von 32 bis 80 mg pro 100 ml Erzeugnis vorliegt.

9. Verwendung der Zusammensetzung nach Anspruch 2, **dadurch gekennzeichnet, dass** Jod in einer Konzentration von 1.600 bis 4.000 mg pro 100 ml Erzeugnis vorliegt.

10. Verwendung der Zusammensetzung nach Anspruch 3, **dadurch gekennzeichnet, dass** Vitamin B5 in einer Konzentration von 48 bis 120 mg pro 100 ml Erzeugnis vorliegt.

11. Verwendung der Zusammensetzung nach Anspruch 3, **dadurch gekennzeichnet, dass** Vitamin B6 in einer Konzentration von 8 bis 20 mg pro 100 ml Erzeugnis vorliegt.

12. Verwendung der Zusammensetzung nach Anspruch 3, **dadurch gekennzeichnet, dass** Vitamin B12 in einer Konzentration von 8 bis 20 mcg pro 100 ml Erzeugnis vorliegt.

13. Verwendung der Zusammensetzung nach Anspruch 4, **dadurch gekennzeichnet, dass** die Aminosäuren, die ausgewählt sind aus der Gruppe bestehend aus Ala/A, Arg/R, Asn/N, Asp/D, Cys/C, Glu/E, Gln/Q, Gly/G, Ile/I, Leu/L, Lys/K, Met/M, Phe/F, Pro/P, Ser/S, Thr/T, Trp/W, Tyr, Y, UND Val/V, in einer Konzentration von 4 bis 10 g pro 100 ml Erzeugnis vorliegen.

14. Verwendung der Zusammensetzung nach Anspruch 6, **dadurch gekennzeichnet, dass** Taurin in einer Konzentration von 4 bis 10 g pro 100 ml Erzeugnis vorliegt.

15. Verwendung der Zusammensetzung nach Anspruch 2, aufweisend die folgende Zusammensetzung für je 100 ml Erzeugnis:
| | |
|---|---|
| Proteine | 5 g |
| Kohlenhydrate | 40,24 g |
| Fette | 0,9 g |
| Arginin | 5 g |
| Vitamin B5 | 60 mg |
| Vitamin B6 | 10 mg |
| Vitamin B12 | 10 mcg |
| Jod | 2.000 mg |
| Phosphoglucan | 40 mg |
| deionisiertes Wasser | nach Bedarf |

16. Sirup für die Behandlung von adenotonsillarer Hypertrophie, aufweisend die folgende Zusammensetzung für je 100 ml Erzeugnis:
| | |
|---|---|
| Fruktose | 28 g |
| Phosphoglucan | 4,4 g |
| Arginin | 5 g |
| Vitamin B5 | 60 mg |
| Vitamin B6 | 10 mg |
| Vitamin B12 | 0,01 mg |
| Kaliumiodid | 26 mg |
| Zitronensäure | 1,8 g |
| Xanthan-Gummi | 70 g |
| Natriumbenzoat | 0,1 g |
| Natrium-Methylparaoxybenzoat | 0,1 g |
| Vanillearoma | 0,35 g |
| Caramel-Farbstoff | 0,01 g |
| deionisiertes Wasser | nach Bedarf |

## Revendications

1. Préparation comprenant de l'arginine, de l'iode, des vitamines du groupe B et un complexe liposomique de CM-glucan (glucane carboxyméthylé).

2. Utilisation d'une composition comprenant de l'arginine, de l'iode, un complexe liposomique de CM-glucan (glucane carboxyméthylé) et des vitamines du groupe B, pour la préparation d'un médicament pour le traitement thérapeutique de l'hypertrophie adénotonsillaire.

3. Utilisation de la composition selon la revendication 2, **caractérisée en ce que** les vitamines du groupe B sont choisies dans le groupe comprenant B5, B6, B12.

4. Utilisation de la composition selon la revendication 2, comprenant en outre des acides aminés choisis dans le groupe comprenant : Ala/A, Arg/R, Asn/N, Asp/D, Cys/C, Glu/E, Gln/Q, Gly/G, Ile/I, Leu/L, Lys/K, Met/M, Phe/F, Pro/P, Ser/S, Thr/T, Trp/W, Tyr/Y et Val/V.

5. Utilisation de la composition selon la revendication 4, **caractérisée en ce que** l'acide aminé est Lys/K.

6. Utilisation de la composition selon l'une quelconque des revendications 2 à 5, comprenant en outre de la taurine.

7. Utilisation de la composition selon la revendication 2, **caractérisée en ce que** l'arginine est dans une concentration de 4 à 10 g par 100 ml de produit.

8. Utilisation de la composition selon la revendication 2, **caractérisée en ce que** le complexe liposomique de CM-glucan (glucane carboxyméthylé) est dans une concentration de 32 à 80 mg par 100 ml de produit.

9. Utilisation de la composition selon la revendication 2, **caractérisée en ce que** l'iode est dans une concentration de 1.600 à 4.000 mg par 100 ml de produit.

10. Utilisation de la composition selon la revendication 3, **caractérisée en ce que** la vitamine B5 est dans une concentration de 48 à 120 mg par 100 ml de produit.

11. Utilisation de la composition selon la revendication 3, **caractérisée en ce que** la vitamine B6 est dans une concentration de 8 à 20 mg par 100 ml de produit.

12. Utilisation de la composition selon la revendication 3, **caractérisée en ce que** la vitamine B12 est dans une concentration de 8 à 20 mcg par 100 ml de produit.

13. Utilisation de la composition selon la revendication 4, **caractérisée en ce que** les acides aminés choisis dans le groupe comprenant Ala/A, Arg/R, Asn/N, Asp/D, Cys/C, Glu/E, Gln/Q, Gly/G, Ile/I, Leu/L, Lys/K, Met/M, Phe/F, Pro/P, Ser/S, Thr/T, Trp/W, Tyr/Y, Val/V a vitamine B5 sont dans une concentration de 4 à 10 g par 100 ml de produit.

14. Utilisation de la composition selon la revendication 6, **caractérisée en ce que** la taurine est dans une concentration de 4 à 10 g par 100 ml de produit.

15. Utilisation de la composition selon la revendication 2, ayant la composition suivante pour chaque 100 ml de produit :
| | |
|---|---|
| Protéines | 5 g |
| Glucides | 40,24 g |
| Graisses | 0,9 g |
| Arginine | 5 g |
| Vitamine B5 | 60 mg |
| Vitamine B6 | 10 mg |
| Vitamine B12 | 10 mcg |
| Iode | 2.000 mg |
| Phospho-glucane | 40 mg |
| Eau dé-ionisée | comme nécessaire |

16. Sirop pour le traitement de l'hypertrophie adénotonsillaire, ayant la composition suivante pour chaque 100 ml de produit :
| | |
|---|---|
| Fructose | 28 g |
| Phospho-glucane | 4,4 g |
| Arginine | 5 g |
| Vitamine B5 | 60 mg |
| Vitamine B6 | 10 mg |
| Vitamine B12 | 0,01 mg |
| Iodure de potassium | 26 mg |
| Acide citrique | 1,8 mg |
| Gomme xanthane | 70 mg |
| Benzoate de sodium | 0,1 g |
| Para-oxybenzoate méthyle-sodium | 0,1 g |
| Vanille naturelle | 0,35 g |
| Caramel colorant | 0,01 g |
| Eau dé-ionisée | comme nécessaire |
